# EUROPEAN PATENT APPLICATION

(11) **EP 4 201 415 A1**
(43) Date of publication of application: **28.06.2023**
(21) Application number: 21856275.9
(22) Date of filing: 12.08.2021
(51) Int. Cl.: A61K 35/74, A61P 1/00, A61P 29/00, A61P 37/08, C12N 1/20

(54) **FAECALIBACTERIUM PRAUSNITZII STRAIN AND USES THEREOF**

(30) Priority: 14.08.2020 KR 20200102564
(71) Applicant: Kobiolabs, Inc., Seoul 08826 (KR)
(72) Inventor: SEO, Boram, Seoul 05409 (KR); JEON, Kyungchan, Uijeongbu-si, Gyeonggi-do 11813 (KR); KIM, Woon Ki, Ulsan 44548 (KR); NAM, Tae Wook, Seongnam-si, Gyeonggi-do 13479 (KR); KIM, Dukyun, Suwon-si, Gyeonggi-do 16330 (KR); KO, Gwang Pyo, Seoul 06732 (KR)
(74) Representative: Grünecker Patent- und Rechtsanwälte PartG mbB
(86) International application number: PCT/KR2021/010768
(87) International publication number: WO 2022/035269

(57) **Abstract**

The present invention relates to a *Faecalibacterium prausnitzii* strain and uses thereof.

## Description

### [TECHNICAL FIELD]

The present invention relates to a *Faecalibacterium prausnitzii* strain and uses thereof.

### [BACKGROUND ART]

About 100 trillion microorganisms, ten times more than the number of cells in the human body, coexist in a healthy human body. The number of their genes is more than 100 times that of humans, and the intestinal microflora is uniquely structured for each individual and disease. A healthy intestinal symbiotic microbiota has complex interactions with human cells and performs a variety of functions, including development of the immune system through regulation of metabolism and immune response, and maintenance of intestinal homeostasis.

### [DISCLOSURE]

### [TECHNICAL PROBLEM]

One embodiment of the present invention is to provide *Faecalibacterium prausnitzii* KBL1027 strain having accession number KCTC14231BP.

Another embodiment of the present invention is to provide a composition, comprising one or more selected from the group consisting of the *Faecalibacterium prausnitzii* strain, a culture of the strain, a lysate of the strain, an extract of the strain, an extract of the culture, an extract of the lysate, or an extracellular polysaccharide produced by the strain.

Other embodiment of the present invention is to provide a use for prevention, improvement or treatment of inflammatory disease of the *Faecalibacterium prausnitzii* strain having therapeutic or prophylactic efficacy of inflammatory disease.

Other embodiment of the present invention is to provide the *Faecalibacterium prausnitzii* strain having anti-inflammatory activity, a culture of the strain, a lysate of the strain, an extract of the strain, an extract of the culture, an extract of the lysate, or an extracellular polysaccharide produced by the strain.

Other embodiment of the present invention is to provide a substance having anti-inflammatory activity, for example, the *Faecalibacterium prausnitzii* strain producing an extracellular polysaccharide, a culture of the strain, a lysate of the strain, an extract of the strain, an extract of the culture, an extract of the lysate, or a substance derived from the strain (for example, extracellular polysaccharide inducing secretion of anti-inflammatory cytokine).

To achieve the above objects, the present invention has discovered novel *Faecalibacterium prausnitzii* KBL1027 strain isolated from healthy Koreans, and has confirmed the excellent efficacy of anti-inflammatory cytokine production and symptom improvement of inflammatory disease, compared to the conventional *Faecalibacterium prausnitzii* strain.

### [TECHNICAL SOLUTION]

One embodiment of the present invention relates to *Faecalibacterium prausnitzii* KBL1027 strain having accession number KCTC14231BP.

Another embodiment of the present invention relates to the *Faecalibacterium prausnitzii* strain having anti-inflammatory activity, a culture of the strain, a lysate of the strain, an extract of the strain, an extract of the culture, an extract of the lysate, or an extracellular polysaccharide produced by the strain.

Other embodiment of the present invention relates to the *Faecalibacterium prausnitzii* strain producing an extracellular polysaccharide, a culture of the strain, a lysate of the strain, an extract of the strain, an extract of the culture, an extract of the lysate, or an extracellular polysaccharide produced by the strain. The extracellular polysaccharide may be a substance having anti-inflammatory activity.

Other embodiment of the present invention relates to a composition, comprising one or more selected from the group consisting of the *Faecalibacterium prausnitzii* strain, a culture of the strain, a lysate of the strain, an extract of the strain, an extract of the culture, an extract of the lysate, and an extracellular polysaccharide produced by the strain.

The composition may be a composition for prevention, improvement, or treatment of inflammatory disease.

The composition may be a food composition or a probiotics composition.

The composition may be a composition for improving the intestinal microbial balance.

Hereinafter, the present invention will be described in more detail.

One embodiment of the present invention relates to *Faecalibacterium prausnitzii* KBL1027 strain having accession number KCTC14231BP.

The strain may be a use for anti-inflammatory, a use for inducing anti-inflammatory cytokine, a use for strengthening tight junction between intestinal epithelial cells, a use for improving the intestinal microbial balance, or a use for prevention, improvement, alleviation or treatment of inflammatory disease.

The *Faecalibacterium prausnitzii* KBL1027 strain may have the 16s rRNA sequence represented by SEQ ID NO: 4.

In the example of the present application, as the result of comparing Average Nucleotide Identity (ANI) of the *Faecalibacterium prausnitzii* KBL1027 strain and *Faecalibacterium prausnitzii* DSM17677 strain belonging to the same species, despite belonging to the same species, they exhibited low average nucleotide identity. This means that the *Faecalibacterium prausnitzii* KBL1027 strain according to one embodiment of the present invention is a novel strain having genetic characteristics distinguished from conventional *Faecalibacterium prausnitzii* microorganisms.

For example, the *Faecalibacterium prausnitzii* KBL1027 strain may have Average Nucleotide Identity (ANI) with the *Faecalibacterium prausnitzii* DSM17677 strain of less than 100%, 99% or less, 98% or less, 97% or less, 96% or less, 95% or less, 94% or less, 93% or less, 92% or less, 91% or less, 90% or less, 89% or less, 88% or less, 87% or less, 86% or less, or 85% or less. Those skilled in the art may clearly perform the *Faecalibacterium prausnitzii* KBL1027 strain having low average nucleotide identity with the *Faecalibacteriumprausnitzii* DSM17677 strain, even though the lower limit of the average nucleotide identity is not specified, and the lower limit of the average nucleotide identity may be 5% or more, 10% or more, 20% or more, 30% or more, 40% or more, 50% or more, 60% or more, 70% or more, or 80%, but not limited thereto.

In an example of the present invention, as the result of comparative genomic analysis of the *Faecalibacterium prausnitzii* KBL1027 strain, compared to the *Faecalibacterium prausnitzii* DSM17677 strain belonging to the same species with the strain, there were a number of genes specific to the *Faecalibacterium prausnitzii* KBL1027. This means that the *Faecalibacterium prausnitzii* KBL1027 strain according to one embodiment of the present invention is a novel strain having genetic characteristics distinguished from conventional *Faecalibacterium prausnitzii* microorganisms.

For example, the *Faecalibacterium prausnitzii* KBL1027 strain may comprise a gene functioning in Cell Wall and Capsule. The gene functioning in Cell Wall and Capsule may perform the function of exopolysaccharide biosynthesis or rhamnose containing glycans. For example, the *Faecalibacterium prausnitzii* KBL1027 strain may comprise one or more genes selected from the group consisting of the following (1) to (7):
(1) Exopolysaccharide biosynthesis glycosyltransferase **EpsF** (EC 2.4.1.-)
(2) Tyrosine-protein kinase **EpsD** (EC 2.7.10.2)
(3) Alpha-D-GlcNAc alpha-1,2-L-rhamnosyltransferase (EC 2.4.1.-) **rgpA**
(4) Alpha-L-Rha alpha-1,3-L-rhamnosyltransferase (EC 2.4.1.-) **rgpB**
(5) capsular polysaccharide biosynthesis protein
(6) Lipoprotein releasing system ATP-binding protein **LolD**
(7) Putative N-acetylgalactosaminyl-diphosphoundecaprenol glucuronosyltransferase **TuaG**

For example, the *Faecalibacterium prausnitzii* KBL1027 strain may comprise one or more genes selected from the group consisting of the following (1) to (13):
(1) Exopolysaccharide biosynthesis glycosyltransferase **EpsF** (EC 2.4.1.-)
(2) Tyrosine-protein kinase **EpsD** (EC 2.7.10.2)
(3) Alpha-D-GlcNAc alpha-1,2-L-rhamnosyltransferase (EC 2.4.1.-) **rgpA**
(4) Alpha-L-Rha alpha-1,3-L-rhamnosyltransferase (EC 2.4.1.-) **rgpB**
(5) capsular polysaccharide biosynthesis protein
(6) Lipoprotein releasing system ATP-binding protein **LolD**
(7) Putative N-acetylgalactosaminyl-diphosphoundecaprenol glucuronosyltransferase **TuaG**
(8) Predicted transcriptional regulator of N-Acetylglucosamine utilization, GntR family **NagQ**
(9) Membrane-bound lytic murein transglycosylase B (EC 3.2.1.-)
(10) Inosine-uridine preferring nucleoside hydrolase (EC 3.2.2.1)
(11) LysR family transcriptional regulator **YnfL**
(12) Cation efflux system protein **CusA**
(13) Cobalt-zinc-cadmium resistance protein **CzcA**

The *Faecalibacterium prausnitzii* KBL1027 strain according to one embodiment of the present invention may have anti-inflammatory characteristics, and specifically, it may have anti-inflammatory characteristics by inducing anti-inflammatory cytokine production.

The *Faecalibacterium prausnitzii* KBL1027 strain according to one embodiment of the present invention may induce anti-inflammatory cytokine production. For example, when LPS and the strain are administered together, the anti-inflammatory cytokine yield (pg/mL), compared to the control group administered with LPS alone may be over 1 time, 1.5 times or more, 2 times or more, 2.5 times or more, 3 times or more, 3.5 times or more, 4 times or more, over 4 times, 4.1 times or more, 4.2 times or more, 4.3 times or more, 4.4 times or more, or 4.5 times or more. The anti-inflammatory cytokine may be IL-10.

The anti-inflammatory cytokine IL-10 is an immunoregulatory cytokine, and is known to play an important role in inflammatory response, oncogenesis, allergy and autoimmune disease. Specifically, IL-10 is an important immunoregulatory cytokine produced in various cells, and it not only inhibits inflammatory response, but also functions to regulate proliferation and differentiation of various immunocytes such as T cells, B cells, NK (natural killer) cells, antigen-presenting cells, mast cells, and the like. IL-10 also exhibits immune activation function to remove infectious or non-infectious particles while minimizing inflammatory response. In addition, IL-10 inhibits antigen-specific activity in most of cells related to allergic response, and inhibits migration of inflammatory cells such as eosinophils, and the like in the inflammatory region, and thereby expression of an allergen, IgE receptor, and the like is reduced, and thus effectively blocks allergen response in monocytes or resin cells. Accordingly, the strain according to one embodiment of the present invention may exhibit the activity to prevent, improve or treat inflammatory disease, allergy or autoimmune disease, or the like, by inducing production of the anti-inflammatory cytokine IL-10.

The *Faecalibacterium prausnitzii* KBL1027 strain according to one embodiment of the present invention may strengthen tight junction between intestinal epithelial cells. For example, when dextran sulfate sodium (DSS) and the strain are administered together, compared to the control group administered with DSS alone, the expression of tight junction-related genes (as one example, *Zo-1* or *Occludin)* may be over 1 time, 1.1 times or more, 1.2 times or more, 1.3 times or more, 1.4 times or more, or 1.5 times or more, or compared to the control group administered with DSS and *Faecalibacterium prausnitzii* DSM17677 strain together, the expression of tight junction-related genes (as one example, *Zo-1* or *Occludin)* may be over 1 time, 1.01 times or more, 1.05 times or more, 1.1 times or more, 1.15 times or more, 1.2 times or more, 1.25 times or more, or 1.3 times or more.

When the tight junction of intestinal epithelial cells is weakened, one kind of immune diseases, intestinal permeability, namely, leaky gut may occur. Specifically, it refers to a phenomenon in which intestinal epithelial cells must establish a protective barrier to induce "tight junction" of the cells to prevent other substances from entering, but foreign substances are introduced and the gut-associated lymphoid tissue (GALT) triggers an immune response to cause inflammation, when there is a problem in the expression and production of tight junction-related proteins. The strain according to one embodiment of the present invention can prevent, improve or treat intestinal permeability or leaky gut by strengthening tight junction of intestinal epithelial cells, and can prevent, improve, alleviate or treat inflammatory response.

The *Faecalibacterium prausnitzii* KBL1027 strain according to one embodiment of the present invention may improve the intestinal microbial balance. When the intestinal microbial imbalance occurs, an abnormal interaction occurs between the host and the microflora, and as a result, immune homeostasis is disrupted and immune-mediated diseases such as autoimmune, allergy and chronic inflammatory diseases may occur. The *Faecalibacterium prausnitzii* KBL1027 strain according to one embodiment of the present invention can prevent, improve, alleviate or treat inflammatory reactions, allergic diseases, autoimmune diseases, and the like, by improving the imbalance of the intestinal microflora in vivo. For example, the *Faecalibacterium prausnitzii* KBL1027 strain may have one or more characteristics selected from the group consisting of the following (1) to (5):
(1) inducing an increase in the diversity of intestinal microflora of an animal,
(2) improving dysbiosis of an animal,
(3) inducing an increase in the relative abundance, or preventing a decrease in the relative abundance of strains of genus Prevotella and/or family Paraprevotellaceae in the intestine of an animal,
(4) preventing an increase in the relative abundance of harmful bacteria, for example, a strain having a lipopolysaccharide cell wall, as one example, a phylum Proteobacteria strain, in intestinal microflora of an animal,
(5) preventing an increase in the relative abundance of a strain of genus Bacteroides in intestinal microflora of an animal.

The *Faecalibacterium prausnitzii* KBL1027 strain according to one embodiment of the present invention may have preventive, improvable or therapeutic activity for inflammatory disease. For example, the *Faecalibacterium prausnitzii* KBL1027 strain may have one or more characteristics selected from the group consisting of the following (1) and (2):
(1) preventing a body weight loss by inflammatory disease, for example, the body weight reduction ratio (Weight change%) is less than 1 time, 0.9 times or less, 0.8 times or less, 0.7 times or less, 0.6 times or less, or 0.5 times or less, when dextran sulfate sodium (DSS) and the strain are administered together, compared to the control group administered with DSS alone,
(2) preventing intestinal length reduction by inflammatory disease, for example, the intestinal length reduction ratio (%) is less than 1 time, 0.9 times or less, 0.8 times or less, 0.7 times or less, 0.6 times or less, 0.5 times or less, 0.4 times or less, 0.3 times or less, 0.2 times or less, or 0.1 time or less, when dextran sulfate sodium (DSS) and the strain are administered together, compared to the control group administered with DSS alone.

Other embodiment of the present invention relates to a composition for preventing, improving or treating inflammatory disease, comprising one or more selected from the group consisting of the *Faecalibacterium prausnitzii* strain, a culture of the strain, a lysate of the strain, an extract of the strain, an extract of the culture, an extract of the lysate, or an extracellular polysaccharide produced by the strain. The *Faecalibacterium prausnitzii* strain may be *Faecalibacteriumprausnitzii* KBL1027 strain having accession number KCTC14231BP.

The inflammatory disease is disease accompanying inflammation, and any disease accompanying inflammation may be included without limitation, and for example, it may be one or more selected from the group consisting of inflammatory bowel disease, inflammatory skin disease, inflammatory collagen-vascular disease, atopic dermatitis, allergic disease, autoimmune disease, autoimmune inflammatory disease, eczema, asthma, allergic asthma, bronchial asthma, rhinitis, allergic rhinitis, conjunctivitis, allergic conjunctivitis, food allergy, rheumatoid arthritis, rheumatoid fever, lupus, systemic scleroderma, psoriasis, psoriatic arthritis, asthma, Guilian-Barre syndrome, myasthenia gravis, dermatomyositis, multiple myositis, multiple sclerosis, autoimmune encephalomyelitis, polyarteritis nodosa, Hashimoto thyroiditis, temporal arteritis, pediatric diabetes, alopecia areata, pemphigus, aphthous stomatitis, autoimmune hemolytic anemia, Wegener granulomatosis, Sjogren syndrome, Addison's disease, Behcet's disease, edema, conjunctivitis, periodontitis, rhinitis, otitis media, chronic sinusitis, sore throat, tonsillitis, bronchitis, pneumonia, stomach ulcer, gastritis, colitis, gout, eczema, acne, contact dermatitis, seborrheic dermatitis, ankylosing myelitis, fibromyalgia, osteoarthritis, shoulder periarthritis, tendinitis, tenosynovitis, myositis, hepatitis, cystitis, nephritis, septicemia, angiitis, and bursitis.

The atopic dermatitis means a chronic or recurrent inflammatory skin condition showing itching.

The allergic disease means that basophils bound to immunoglobulin E (IgE) produced by the immune system exposed to an antigen and histamine secreted by mast cells induce inflammation of surrounding tissues.

The autoimmune disease means that an immune response to a substance in the body has occurred and an inflammatory response by autoimmunity has occurred in an organ or tissue. The autoimmune disease may be for example, inflammatory skin disease. The inflammatory skin disease may be for example, one or more selected from the group consisting of atopy, psoriasis, eczema, acne, contact dermatitis and seborrheic dermatitis. The autoimmune disease may be local autoimmune disease or systemic autoimmune disease. The local autoimmune disease may be for example, one or more selected from the group consisting of type 1 diabetes, hypothyroidism, hyperthyroidism, idiopathic thrombocytopenia, and leukoplakia, but not limited thereto. The systemic autoimmune disease may be one or more selected from the group consisting of systemic erythematosus lupus, rheumatoid arthritis, Sjogren syndrome, Behect's disease, systemic sclerosis, multiple myositis, and dermatomyositis, but not limited thereto.

The inflammatory bowel disease (IBD) is inflammatory disease of the gastrointestinal tract, and the cause is often unknown, and research on drug development to treat the inflammatory bowel disease has been enormously carried out, and as the result of analyzing the intestinal microflora of patients with inflammatory bowel disease and ulcerative colitis using the next-generation sequencing method, there was dysbiosis as the diversity of the intestinal microflora decreases and Proteobacteria dominates in the patients compared to healthy normal people.

The inflammatory bowel disease may be one or more selected from the group consisting of inflammatory colorectal disease, ulcerative colitis (UC), enteritis, irritable bowel syndrome (IBS), and Crohn's disease (CD).

The inflammatory disease according to one embodiment of the present invention may cause one or more symptoms or pathological phenomena selected from the group consisting of the following (1) to (10):
(1) inducing inflammatory cytokine,
(2) inhibition of anti-inflammatory cytokine,
(3) hyperactivation of autoimmunity,
(4) body weight loss of an animal,
(5) intestinal length reduction of an animal,
(6) weakening tight junction of intestinal epithelial cells of an animal,
(7) dysbiosis of an animal,
(8) increase of relative abundance of harmful bacteria, for example, a strain having a lipopolysaccharide cell wall, as one example, a phylum Proteobacteria strain, in the intestinal microflora of an animal,
(9) increase of relative abundance of genus *Bacteroides* strain in the intestinal microflora of an animal,
(10) decrease of relative abundance of genus *Prevotella* and/or family Paraprevotellaceae strains in the intestinal microflora of an animal.

The composition according to one embodiment of the present invention may have an anti-inflammatory characteristic, and specifically, it may have an anti-inflammatory characteristic by inducing anti-inflammatory cytokine production. For example, when LPS and the composition are administered together, compared to the control group administered with LPS alone, the anti-inflammatory cytokine production (pg/mL) may be over 1 time, 1.5 times or more, 2 times or more, 2.5 times or more, 3 times or more, 3.5 times or more, 4 times or more, over 4 times, 4.1 times or more, 4.2 times or more, 4.3 times or more, 4.4 times or more, or 4.5 times or more. The anti-inflammatory cytokine may be IL-10.

The composition according to one embodiment of the present invention may have a characteristic of strengthening tight junction between intestinal epithelial cells of an animal. For example, when dextran sulfate sodium (DSS) and the composition are administered together, compared to the control group administered with DSS alone, the expression of a tight junction-related gene (as one example, *Zo-1* or *Occludin)* may be over 1 time, 1.1 times or more, 1.2 times or more, 1.3 times or more, 1.4 times or more, or 1.5 times or more, or, compared to the control group administered with DSS and *Faecalibacterium prausnitzii* DSM17677 together, the expression of a tight junction-related gene (as one example, *Zo-1* or *Occludin)* may be over 1 time, 1.01 times or more, 1.05 times or more, 1.1 times or more, 1.15 times or more, 1.2 times or more, 1.25 times or more, or 1.3 times or more.

The composition according to one embodiment of the present invention may improve the intestinal microbial balance. For example, the composition may have one or more characteristics selected from the group consisting of the following (1) to (5):
(1) inducing an increase in the diversity of intestinal microflora of an animal,
(2) improving dysbiosis of an animal,
(3) preventing an increase in the relative abundance of harmful bacteria, for example, a strain having a lipopolysaccharide cell wall, as one example, a phylum Proteobacteria strain, in intestinal microflora of an animal,
(4) preventing an increase in the relative abundance of a strain of genus *Bacteroides* in intestinal microflora of an animal,
(5) inducing an increase in the relative abundance or preventing a decrease in the relative abundance of strains of genus *Prevotella* and/or family Paraprevotellaceae in the intestine of an animal,

The composition according to one embodiment of the present invention may have preventive, improvable or therapeutic activity of inflammatory disease. For example, the composition may have one or more characteristics selected from the group consisting of the following (1) to (2):
(1) preventing a body weight loss of an animal by inflammatory disease, for example, the body weight reduction ratio (Weight change%) is less than 1 time, 0.9 times or less, 0.8 times or less, 0.7 times or less, 0.6 times or less, or 0.5 times or less, when dextran sulfate sodium (DSS) and the composition are administered together, compared to the control group administered with DSS alone,
(2) preventing intestinal length reduction of an animal by inflammatory disease, for example, the intestinal length reduction ratio (%) is less than 1 time, 0.9 times or less, 0.8 times or less, 0.7 times or less, 0.6 times or less, 0.5 times or less, 0.4 times or less, 0.3 times or less, 0.2 times or less, or 0.1 time or less, when dextran sulfate sodium (DSS) and the composition are administered together, compared to the control group administered with DSS alone.

The composition may improve the intestinal microbial balance. For example, the composition may increase the diversity of the intestinal microflora of an animal. As one example, the composition may increase the relative abundance of strains of genus *Prevotella* and/or family Paraprevotellaceae in the intestine of an animal. As one example, the composition may decrease the relative abundance of strains of genus *Bacteroides* and/or phylum Proteobacteria in the intestine of an animal.

The composition may comprise an extracellular polysaccharide of a *Faecalibacterium prausnitzii* strain. According to the example of the present application, it was confirmed that the *Faecalibacterium prausnitzii* KBL1027 strain specifically produces an extracellular polysaccharide, and thus the composition may comprise an extracellular polysaccharide of the *Faecalibacteriumprausnitzii* KBL1027 strain having accession number KCTC14231BP.

It was confirmed that the *Faecalibacterium prausnitzii* KBL1027 strain according to the present invention has excellent efficacy to improve symptoms of colitis disease compared to other strains belonging to the same species based on experiments, and the effects of strengthening the expression of genes of anti-inflammatory cytokine *IL-10* and *Zo-1* and *Occludin* related to tight junction function between intestinal epithelial cells, and recovery of intestinal microflora have been confirmed. In addition, an extracellular polysaccharide substance specifically produced by the *Faecalibacterium prausnitzii* KBL1027 strain was confirmed experimentally and genomically, and the productivity of anti-inflammatory cytokine IL-10 by culture solution of *Faecalibacterium prausnitzii* KBL 1027 strain was confirmed on the basis of BMDM. Such results mean that the *Faecalibacterium prausnitzii* KBL1027 strain can be usefully used for prevention, improvement and treatment of inflammatory disease.

Other embodiment of the present invention relates to a *Faecalibacterium prausnitzii* strain having anti-inflammatory activity, a culture of the strain, a lysate of the strain, an extract of the strain, an extract of the culture, an extract of the lysate or an extracellular polysaccharide produced by the strain. The *Faecalibacterium prausnitzii* strain may be *Faecalibacterium prausnitzii* KBL1027 strain having accession number KCTC14231BP.

Other embodiment of the present invention relates to a *Faecalibacterium prausnitzii* strain producing a substance having anti-inflammatory activity, for example, an extracellular polysaccharide, a culture of the strain, a lysate of the strain, an extract of the strain, an extract of the culture, an extract of the lysate, or an extracellular polysaccharide produced by the strain. The *Faecalibacterium prausnitzii* strain may be *Faecalibacterium prausnitzii* KBL1026 strain having accession number KCTC14230BP or *Faecalibacterium prausnitzii* KBL1027 strain having accession number KCTC14231BP, and preferably, it may be *Faecalibacterium prausnitzii* KBL1027 strain having accession number KCTC14231BP.

Other embodiment of the present invention relates to a food composition, comprising one or more selected from the group consisting of a *Faecalibacterium prausnitzii* strain, a culture of the strain, a lysate of the strain, an extract of the strain, an extract of the culture, an extract of the lysate, or an extracellular polysaccharide produced by the strain. The *Faecalibacterium prausnitzii* strain is as described above. As one example, the *Faecalibacterium prausnitzii* strain may be *Faecalibacterium prausnitzii* KBL1027 strain having accession number KCTC14231BP.

Other embodiment of the present invention relates to a probiotics composition, comprising one or more selected from the group consisting of a *Faecalibacterium prausnitzii* strain, a culture of the strain, a lysate of the strain, an extract of the strain, an extract of the culture, an extract of the lysate, or an extracellular polysaccharide produced by the strain. The *Faecalibacterium prausnitzii* strain is as described above. As one example, the *Faecalibacterium prausnitzii* strain may be *Faecalibacterium prausnitzii* KBL1027 strain having accession number KCTC14231BP.

Other embodiment of the present invention relates to a composition for improving the intestinal microbial balance, comprising one or more selected from the group consisting of a *Faecalibacterium prausnitzii* strain, a culture of the strain, a lysate of the strain, an extract of the strain, an extract of the culture, an extract of the lysate, or an extracellular polysaccharide produced by the strain. The *Faecalibacterium prausnitzii* strain is as described above. As one example, the *Faecalibacterium prausnitzii* strain may be *Faecalibacterium prausnitzii* KBL1027 strain having accession number KCTC14231BP.

Other embodiment of the present invention relates to a method for prevention, improvement or treatment of inflammatory disease, comprising administering one or more selected from the group consisting of a *Faecalibacterium prausnitzii* KBL1027 strain having accession number KCTC1423 1BP, a culture of the strain, a lysate of the strain, an extract of the strain, an extract of the culture, an extract of the lysate, or an extracellular polysaccharide produced by the strain. The *Faecalibacterium prausnitzii* KBL1027 strain, the inflammatory disease, and the like are as described above.

Other embodiment of the present invention relates to a method for improving the intestinal microbial balance, comprising administering one or more selected from the group consisting of a *Faecalibacterium prausnitzii* KBL1027 strain having accession number KCTC14231BP, a culture of the strain, a lysate of the strain, an extract of the strain, an extract of the culture, an extract of the lysate, or an extracellular polysaccharide produced by the strain. In the example of the present application, as the result of administering the strain according to one embodiment of the present invention, an increase of the diversity of the intestinal microflora was induced, and the dysbiosis of an animal was improved, and specifically, an increase of the relative abundance of harmful bacteria, for example, a strain having a lipopolysaccharide cell wall, as one example, a strain of phylum Proteobacteria is prevented in the intestinal microflora of an animal, and an increase of the relative abundance of a strain of genus *Bacteroides* in the intestinal microflora of an animal was prevented, and an increase of the relative abundance of strains of genus *Prevotella* and/or family Paraprevotellaceae was induced, or a decrease of the relative abundance was prevented, in the intestinal microflora of an animal. Accordingly, the intestinal microbial balance of an animal may be improved by administering the strain according to one embodiment of the present invention.

Herein, the term `active ingredient' means an ingredient which exhibits desired activity alone or may exhibit activity together with a carrier having no activity by itself.

Herein, the term 'prevention' means inhibiting or delaying occurrence of illness, disorder or illness. Prevention may be considered complete, when the occurrence of illness, disorder or disease is inhibited or delayed for a predetermined period of time.

Herein, the term 'treatment' means lightening, improving, alleviating, inhibiting or delaying specific illness, disorder and/or disease or symptoms caused by disease partially or completely, and reducing the severity, or reducing occurrence of one or more symptoms or characteristics.

The composition, for example, pharmaceutical composition of the present invention may further comprise one or more active ingredients showing the same or similar function in addition to the above active ingredient.

Furthermore, the composition, for example, pharmaceutical composition according to the present invention may be prepared in a unit dose form by formulating using a pharmaceutically acceptable carrier or be prepared by being included in a high-dose container, according to a method which may be clearly conducted by those skilled in the art to which the invention pertains. Herein, the term 'carrier' means a compound which allows addition of a compound into a cell or tissue easily, and the term `pharmaceutically acceptable' refers to a composition which is physiologically acceptable and when administered into a human, commonly, does not cause allergic response such as gastrointestinal disorder and dizziness or response similar thereto.

The pharmaceutically acceptable carrier is commonly used, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil, and the like, but not limited thereto.

In addition, the composition, for example, pharmaceutical composition according to the present invention may further comprise an additive such as a filler, an anti-coagulant, a lubricant, a wetting agent, a flavoring, an emulsifier, a preservative, and the like, in addition to the above ingredients. Herein, the content of the additive comprised in the composition is not particularly limited, and may be appropriately adjusted in the content range used for common formulation.

Furthermore, the composition, for example, pharmaceutical composition, food composition or probiotics composition according to the present invention may be formulated as an oral formulation. The non-limitative examples of the oral formulation include tablets, troches, lozenge, aqueous suspension, oily suspension, prepared powder, granules, emulsion, hard capsules, soft capsules, syrup or elixirs, or the like. To formulate the pharmaceutical composition according to the present invention for oral administration, a binding agent such as lactose, saccharose, sorbitol, mannitol, starch, amylopectin, cellulose or gelatin; an excipient such as dicalcium phosphate; a disintegrating agent such as corn starch or sweet potato starch; magnesium stearate, calcium stearate, sodium stearyl fumarate, and the like may be used, and a sweetener, an aromatic, syrup and the like may be used. Moreover, in case of capsules, in addition to the aforementioned substances, a liquid carrier such as fat oil, and the like may be additionally used.

Herein, the term 'excipient' means any substance other than a therapeutic agent, and means one used or added to a pharmaceutical composition as a carrier or medium for delivery of a therapeutic agent. Thereby, it may improve handling and storage characteristics or allow and facilitate unit dose formation of the composition.

The composition, for example, pharmaceutical composition, food composition or probiotics composition according to the present invention may be used by formulating in various forms such as oral formulations including liquids, suspensions, powder, granules, tablets, capsules, pills, extract, emulsion, syrup, aerosol, injections of sterile injection solution, and the like, and it may be administered through various routes including oral administration or intravenous, intraperitoneal, subcutaneous, rectal, local administration, and the like. Herein, the term `oral administration' means that a substance prepared to make an active substance be ingested, that is, is administered to the gastrointestinal tract for absorption.

A preferable dose of the composition, for example, pharmaceutical composition, food composition or probiotics composition according to the present invention may vary in its range depending on the patient's condition and body weight, age, gender, health condition, dietary constitutional specificity, formulation properties, degree of disease, composition administration time, administration method, administration period or interval, excretion rate and drug type, and may be appropriately selected by those skilled in the art.

Herein, the term `effective dose of pharmaceutical composition' means an amount of a composition of sufficient active ingredients to treat specific symptoms. This may vary by the formulation method, administration method, administration time and/or administration route, and the like, of the pharmaceutical composition, and it may vary depending on various factors including the type and degree of response to be achieved by administration of the pharmaceutical composition, the kind, age, body weight, common health condition, symptoms or severity of disease, gender, diet, excretion, of subject to be administered, components of drug other compositions used simultaneously or at the same time together, and similar factors well known in the pharmaceutical field, and those skilled in the art may readily determine and prescribe an effective dose for desired treatment.

The administration of the pharmaceutical composition according to the present invention may be administered once a day or may be administered divided into several times. The composition may be administered as an individual therapeutic agent or administered in combination with other therapeutic agents, and may be administered sequentially or simultaneously with conventional therapeutic agents. Considering all of the above factors, it may be administered in an amount that can obtain the maximum effect with a minimum amount without side effects.

For example, the composition according to the present invention may be administered in a daily dose of 0.001 to 10,000 mg, 0.001 to 5,000 mg, 0.001 to 1,000 mg, 0.001 to 500 mg, 0.001 to 300 mg, 0.001 to 100 mg, 0.001 to 50 mg, 0.001 to 30 mg, 0.001 to 10 mg, 0.001 to 5 mg, 0.001 to 1 mg, 0.001 to 0.5 mg, 0.001 to 0.1 mg, 0.001 to 0.05 mg, 0.001 to 0.01 mg, 0.01 to 10,000 mg, 0.01 to 5,000 mg, 0.01 to 1,000 mg, 0.01 to 500 mg, 0.01 to 300 mg, 0.01 to 100 mg, 0.01 to 50 mg, 0.01 to 30 mg, 0.01 to 10 mg, 0.01 to 5 mg, 0.01 to 1 mg, 0.01 to 0.5 mg, 0.01 to 0.1 mg, 0.01 to 0.05 mg, 0.1 to 10,000 mg, 0.1 to 5,000 mg, 0.1 to 1,000 mg, 0.1 to 500 mg, 0.1 to 300 mg, 0.1 to 200 mg, 0.1 to 100 mg, 0.1 to 50 mg, 0.1 to 30 mg, 0.1 to 10 mg, 0.1 to 5 mg, 0.1 to 1 mg, 0.1 to 0.5 mg, 1 to 10,000 mg, 1 to 5,000 mg, 1 to 1,000 mg, 1 to 500 mg, 1 to 300 mg, 1 to 200 mg, 1 to 100 mg, 1 to 50 mg, 1 to 10 mg, 1 to 5 mg, 10 to 10,000 mg, 10 to 5,000 mg, 10 to 1,000 mg, 10 to 500 mg, 10 to 300 mg, 10 to 200 mg, 10 to 100 mg, 10 to 50 mg, 10 to 40 mg, 10 to 30 mg, 10 to 20 mg, 100 to 10,000 mg, 100 to 5,000 mg, 100 to 1,000 mg, 100 to 500 mg, 100 to 300 mg, or 100 to 200 mg per body weight 1 kg, but not limited thereto. As one example, the daily dose of the composition according to the present invention may be 0.001 to 10 g/1 day, 0.001 to 5 g/1 day, 0.01 to 10 g/1 day, or 0.01 to 5 g/1 day based on oral administration of an adult patient. In addition, the total daily dose may be divided and administered continuously or non-continuously if necessary.

Herein, the term "culture of strain" means products obtained after culturing the strain according to one embodiment of the present invention, and the culture may be total cultures of the strain according to one embodiment of the present invention, diluted solution, concentrates, dried materials, freeze-dried materials, lysates and/or fractions, and the like, and the concentrates may be obtained by centrifuging or evaporating the culture, and the dried materials may be obtained by drying the culture using a drier, or the like, and the freeze-dried materials may be obtained by freeze-drying using a freeze dryer, or the like, and the lysates may be obtained physically or by sonicating the strain or culture, and the fractions may be obtained by applying the culture, lysate, and the like to a method such as centrifugation, chromatography, and the like. The culture may be a solid phase (solid, for example, dried materials), liquid phase (liquid) or fluidized phase, but not limited thereto. In one example, the culture may mean a total medium comprising a cultured strain obtained by culturing the strain according to one embodiment of the present invention for a certain period, metabolites thereof and/or extra nutrients, and the like. In one example, the culture may be one in which the strain according to one embodiment of the present invention is removed or not removed. In one example, the culture may mean the remaining components except the strain (microbial cells) in the culture that the strain according to one embodiment of the present invention is cultured in a medium. In one example, the culture may be a culture solution (or culture) that the strain (microbial cells) is removed in the culture solution that the strain according to one embodiment of the present invention is cultured in a medium. The culture solution (or culture) in which the strain is removed may be a cell free culture solution (or culture) or culture solution comprising dead cells, and for example, it may be a filtrate in which the strain is removed by filtration or centrifugation (centrifuged supernatant) and/or culture solution (or dried materials of the culture solution) comprising dead cells. Specifically, the culture may exhibit anti-inflammatory activity or preventive, improvable or therapeutic activity of inflammatory disease, at the equivalent level to the activity shown by the strain according to one embodiment of the present invention. As one example, the culture may comprise an extracellular polysaccharide produced by the strain according to one embodiment of the present invention.

Herein, the term "lysate of strain" may mean products obtained by lysing the strain according to one embodiment of the present invention by chemical or physical power. Specifically, the lysate may exhibit anti-inflammatory activity or preventive, improvable or therapeutic activity of inflammatory disease, at the equivalent level to the activity shown by the strain according to one embodiment of the present invention. As one example, the lysate may comprise an extracellular polysaccharide produced by the strain according to one embodiment of the present invention.

Herein, the term "extract" may mean products obtained by extracting the strain according to one embodiment of the present invention, a culture of the strain, a lysate of the strain or a mixture thereof, irrespective of the extraction method, extraction solvent, extracted component or form of the extract, and is a broad concept that includes all substances that may be obtained by processing or treating by other methods after extraction. For example, the extract may be an extract of the strain according to one embodiment of the present invention, an extract of the culture of the strain, or an extract of the lysate of the strain. Specifically, the extract may exhibit anti-inflammatory activity or preventive, improvable or therapeutic activity of inflammatory disease, at the equivalent level to the activity shown by the strain according to one embodiment of the present invention, a culture of the strain or a lysate of the strain. As one example, the extract may comprise an extracellular polysaccharide produced by the strain according to one embodiment of the present invention.

### [ADVANTAGEOUS EFFECTS]

The *Faecalibacterium prausnitzii* strain according to the one embodiment of the present invention has excellent anti-inflammatory cytokine production promotion compared to the conventional strain, and thus exhibits anti-inflammatory properties, thereby exhibiting a therapeutic effect on inflammatory disease, and therefore it may be used in a composition for prevention, improvement and treatment of inflammatory disease.

### [BRIEF DESCRIPITON OF THE DRAWINGS]

FIG. 1a is a drawing which shows the result of intestinal microflora analysis of K0-16 subjects from which *Faecalibacteriumprausnitzii* KBL1027 strain is isolated and K0-13 subjects from which KBL1026 strain is isolated, based on 16S rRNA.
FIG. 1b is a drawing which shows the result of intestinal microflora analysis of K0-16 subjects from which *Faecalibacteriumprausnitzii* KBL1027 strain is isolated and K0-13 subjects from which KBL1026 strain is isolated.
FIG. 2a is a drawing which shows the body weight change according to the administration of the *Faecalibacterium prausnitzii* strain according to one embodiment of the present invention.
FIG. 2b is a drawing which shows the colon length change according to the administration of the *Faecalibacterium prausnitzii* strain according to one embodiment of the present invention.
FIG. 2c is a drawing which shows the process of measuring the colon length change according to the administration of the *Faecalibacterium prausnitzii* strain according to one embodiment of the present invention.
FIG. 3 is a drawing which shows the H&E staining result after the administration of the *Faecalibacterium prausnitzii* strain according to one embodiment of the present invention.
FIG. 4a is a drawing which shows the change in expression of inflammatory cytokine gene IL-10 according to the administration of the *Faecalibacterium prausnitzii* strain according to one embodiment of the present invention.
FIG. 4b is a drawing which shows the change in expression of tight junction gene Zo-1 according to the administration of the *Faecalibacterium prausnitzii* strain according to one embodiment of the present invention.
FIG. 4c is a drawing which shows the change in expression of tight junction gene Occludin according to the administration of the *Faecalibacterium prausnitzii* strain according to one embodiment of the present invention.
FIG. 5a is a drawing which shows the change in the diversity of the intestinal microflora according to the administration of the *Faecalibacterium prausnitzii* strain according to one embodiment of the present invention.
FIG. 5b is a drawing which shows the result of analysis of major components through weighted UniFrac distance of intestinal microflora after the administration of the *Faecalibacterium prausnitzii* strain according to one embodiment of the present invention.
FIG. 5c is a drawing which shows the result of univariate analysis (LefSE) to investigate the change in intestinal microflora according to the administration of the *Faecalibacterium prausnitzii* strain according to one embodiment of the present invention.
FIG. 6 is a drawing which shows the result of observing the *Faecalibacterium prausnitzii* strain with a scanning electron microscope.
FIG. 7 is a drawing which shows the inflammatory efficacy by an extracellular polysaccharide specifically produced by the *Faecalibacterium prausnitzii* KBL1027 strain.
FIG. 8 is a drawing which shows genes specifically present in the genome of the *Faecalibacterium prausnitzii* KBL1027 strain according to their functional characteristics.
FIG. 9 is a drawing which shows genes belonging to the Cell Wall and Capsule functions specifically present in the genome of the *Faecalibacterium prausnitzii* KBL1027 strain.

### [MODE FOR INVENTION]

Hereinafter, the present invention will be described in more detail by the following examples. However, these examples are intended to illustrate the present invention only, but the scope of the present invention is not limited by these examples.

### Example 1. Isolation of novel strains

From the intestinal microflora of Koreans, novel *Faecalibacterium prausnitzii* strains were isolated. Specifically, a sample for isolating intestinal microflora was isolated from feces samples provided from healthy normal adults different each other who did not take an antibiotic for 6 months (Seoul National University IRB approval number: 1602/001-001). The feces samples were transported to the present laboratory and moved to an anaerobic chamber (Laboratory Products Inc.) immediately to use for isolation of strains. The samples were streaked in a YCFAG medium comprising 1.5% agar in a direct smearing form, and then were cultured under an anaerobic condition at 37 °C for 48 hours at maximum. After culturing, a purely isolated colony was randomly selected and cultured in a YBHI medium, and for a long-term storage of the strains, glycerol (25 % v/v) was added to the culture solution which reached the exponential phase and was stored in a -80 °C ultralow temperature freezer. For identification of the strains, the genomic DNA of strains were extracted using Wizard genomic purification kit (Promega) and then PCR reaction was performed using 27F/1492R primers (SEQ ID NOs: 1 and 2) of the following Table 1 using a 16S rRNA gene as a target.

**[Table 1]**

| Classification | Sequence (5'→3') | SEQ ID NO: |
|---|---|---|
| Forward | AGAGTTTGATYMTGGCTCAG | 1 |
| Reverse | TACGGYTACCTTGTTACGACT | 2 |

This was purified using QIAquick PCR purification kit (Qiagen), and then sequencing was conducted using ABI3711 automatic sequencer (Macrogen). The result of 16S rRNA analysis was as the following Table 2, and the identification of the strains was finally completed by multiple comparison with EzBioCloud program of Chunlab (http://www.ezbiocloud.net/identify) using this sequence information.

**[Table 2]**

| Strain name | Name | Sequence | SEQ ID NO: |
|---|---|---|---|
| *Faecalibacterium prausnitzii* | KBL 1026 | GACGAACGCTGGCGGCGCGCCTAACACATGCAAGTCGAA CGAGTGAGAGAGAGCTTGCTTTCTCGAGCGAGTGGCGAA CGGGTGAGTAACGCGTGAGGAACCTGCCTCAAAGAGGGG GACAACAGTTGGAAACGACTGCTAATACCGCATAAGCCC ACGACCCGGCATCGGGTAGAGGGAAAAGGAGCAATCCG CTTTGAGATGGCCTCGCGTCCGATTAGCTAGTTGGTGAGG TAACTGGCCCACCAAGGCGACGATCGGTAGCCGGACTGA GAGGTTGAACGGCCACATTGGGACTGAGACACGGCCCAG ACTCCTACGGGAGGCAGCAGTGGGGAATATTGCACAATG GGGGAAACCCTGATGCAGCGACGCCGCGTGGAGGAAGA AGGTCTTCGGATTGTAAACTCCTGTTGTTGAGGAAGATAA TGACGGTACTCAACAAGGAAGTGACGGCTAACTACGTGC CAGCAGCCGCGGTAAAACGTAGGTCACAAGCGTTGTCCG GAATTACTGGGTGTAAAGGGAGCGCAGGCGGGAAGACA AGTTGGAAGTGAAATCCATGGGCTCAACCCATGAACTGC TTTCAAAACTGTTTTTCTTGAGTAGTGCAGAGGTAGGCGG AATTCCCGGTGTAGCGGTGGAATGCGTAGATATCGGGAG GAACACCAGTGGCGAAGGCGGCCTACTGGGCACCAACTG ACGCTGAGGCTCGAAAGTGTGGGTAGCAAACAGGATTAG ATACCCTGGTAGTCCACACTGTAAACGATGATTACTAGGT GTTGGAGGATTGACCCCTTCAGTGCCGCAGTTAACACAAT AAGTAATCCACCTGGGGAGTACGACCGCAAGGTTGAAAC TCAAAGGAATTGACGGGGGCCCGCACAAGCAGTGGAGTA TGTGGTTTAATTCGACGCAACGCGAAGAACCTTACCAAG TCTTGACATCCTGCGACGCACATAGAAACAGTAGTTTCCT TCGGGACGCAGAGACAGGTGGTGCATGGTTGTCGTCAGC TCGTGTCGTGAGATGTTGGGTTAAGTCCCGCAACGAGCG CAACCCTTATGGTCAGTTACTACGCAAGAGGACTCTGGCC AGACTGCCGTTGACAAAACGGAGGAAGGTGGGGATGAC GTCAAATCATCATGCCCTTTATGACTTGGGCTACACACGT ACTACAATGGCGTTAAACAAAGAGAAGCAAGACCGCGA GGTGGAGCAAAACTCAGAAACAACGTCCCAGTTCGGACT GCAGGCTGCAACTCGCCTGCACGAAGTCGGAATTGCTAG TAATCGCAGATCAGCATGCTGCGGTGAATACGTTCCCGG GCCTTGTACACACCGCCCGTCACACCATGAGAGCCGGGG GGACCCGAAGTCGGT | 3 |
| *Faecalibacterium prausnitzii* | KBL1027 | TGAATTTGGCTCAGGACGAACGCTGGCGGCGCGCCTAAC ACATGCAAGTCGAACGAGCGAGAGAGAGCTTGCTTTCTC AGAGCGAGTGGCGAACGGGTGAGTAACGCGTGAGGAAC CTGCCTCAAAGAGGGGGACAACAGTTGGAAACGACTGCT | 4 |
| | | AATACCGCATAAGCCCACGACCCGGCATCGGGTAGAGGG AAAAGGAGCAATCCGCTTTGAGATGGCCTCGCGTCCGAT TAGCTAGTTGGTGAGGTAACGGCCCACCAAGGCGACGAT CGGTAGCCGGACTGAGAGGTTGAACGGCCACATTGGGAC TGAGACACGGCCCAGACTCCTACGGGAGGCAGCAGTGGG GAATATTGCACAATGGGGGAAACCCTGATGCAGCGACGC CGCGTGGAGGAAGAAGGTCTTCGGATTGTAAACTCCTGT TGTTGAGGAAGATAATGACGGTACTCAACAAGGAAGTGA CGGCTAACTACGTGCCAGCAGCCGCGGTAAAACGTAGGT CACAAGCGTTGTCCGGAATTACTGGGTGTAAAGGGAGCG CAGGCGGGAAGACAAGTTGGAAGTGAAATCCATGGGCTC AACCCATGAACTGCTTTCAAAACTGTTTTTCTTGAGTAGT GCAGAGGTAGGCGGAATTCCCGGTGTAGCGGTGGAATGC GTAGATATCGGGAGGAACACCAGTGGCGAAGGCGGCCTA CTGGGCACCAACTGACGCTGAGGCTCGAAAGTGTGGGTA GCAAACAGGATTAGATACCCTGGTAGTCCACACTGTAAA CGATGATTACTAGGGGTTGGAGGATTGACCCCTTCAGTGC CGCAGTTAACACAATAAGTAATCCACCTGGGGAGTACGA CCGCAAGGTTGAAACTCAAAGGAATTGACGGGGGCCCGC ACAAGCAGTGGAGTATGTGGTTTAATTCGACGCAACGCG AAGAACCTTACCAAGTCTTGACATCCTGCGACGCACATA GAAATATGTGTTTCCTTCGGGACGCAGAGACAGGTGGTG CATGGTTGTCGTCAGCTCGTGTCGTGAGATGTTGGGTTAA GTCCCGCAACGAGCGCAACCCTTATGGTCAGTTACTACGC AAGAGGACTCTGGCCAGACTGCCGTTGACAAAACGGAGG AAGGTGGGGATGACGTCAAATCATCATGCCCTTTATGACT TGGGCTACACACGTACTACAATGGCGTTAAACAAAGAGA AGCAAGACCGCGAGGTGGAGCAAAACTCAGAAACAACG TCCCAGTTCGGACTGCAGGCTGCAACTCGCCTGCACGAA GTCGGAATTGCTAGTAATCGCAGATCAGCATGCTGCGGT GAATACGTTCCCGGGCCTTGTACACACCGCCCGTCACACC ATGAGAGCCGGGGGGACCCGAAGTCGGTAGTCTAACCGC AAGGAG | |

Two kinds of novel strains isolated were named *Faecalibacterium prausnitzii* KBL1026 strain and *Faecalibacterium prausnitzii* KBL1027 strain, respectively, and deposited to Korea Research Institute of Bioscience and Biotechnology Biological Resource Center on July 7, 2020, and received accession numbers KCTC14230BP (Faecalibacterium prausnitzii KBL1026) and KCTC14231BP (Faecalibacterium prausnitzii KBL1027), respectively.

### Example 2. Analysis of structure of intestinal microflora of subjects with novel strains

Novel strains isolated in Example 1, the *Faecalibacterium prausnitzii* KBL1026 strain was derived from K0-13 subject and the *Faecalibacterium prausnitzii* KBL1027 strain was derived from K0-16. To analyze the structure of the intestinal microflora of subjects with novel strains, the secured feces samples were stored in a -80°C ultralow temperature freezer for the purpose of intestinal microflora analysis, and the frozen samples were transferred to the laboratory and the total bacterial genomic DNA in feces was extracted using QIAamp FAST DNA stool mini kit (Qiagen). The extracted DNA was amplified using 515F/806R primers (SEQ ID NOs: 5 and 6) of the following Table 3 targeting V4 region of the 16S rRNA gene of bacteria, and then sequence data were generated using MiSeq (Illumina). In the generated large-capacity sequencing analysis, the structure of the intestinal flora was identified by confirming the entire genetic information of the intestinal bacteria using QIIME pipeline.

**[Table 3]**

| Classification | Sequence (5'→3') | SEQ ID NO: |
|---|---|---|
| Forward | ATGATACGGCGACCACCGAGATCTACACTATGGTAATTGTGTGCCA GCMGCCGCGGTAA | 5 |
| Reverse | CAAGCAGAAGACGGCATACGAGATAGTCAGTCAGCCGGACTACHV GGGTWTCTAAT | 6 |

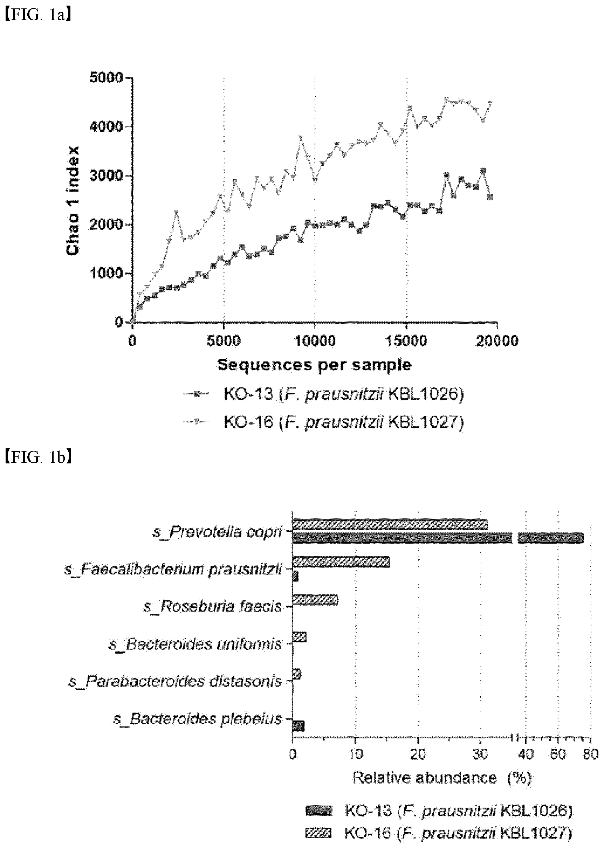

As the result of analyzing the intestinal microflora based on 16S rRNA, as shown in FIG. 1a, it was confirmed that the K0-16 subject in which the *Faecalibacterium prausnitzii* KBL1027 strain was isolated, had the high diversity of the intestinal microflora than the K0-13 subject in which the KBL1026 strain was isolated (FIG. 1a).

In addition, as the result of analyzing the distribution of bacteria accounting for the ratio of 1% or more at a species level of the microflora, as shown in FIG. 1b, it was confirmed that the K0-16 subj ect was highly dominant as the relative abundance of the *Faecalibacterium prausnitzii* strain in the intestine was 15.5% or more (FIG. 1b). On the other hand, it was confirmed that the K0-13 subject in which the KBL1026 strain was isolated accounted for a very low ratio as the relative abundance of the *Faecalibacterium prausnitzii* strain in the intestine was 0.8% (FIG. 1b).

This difference of the *Faecalibacterium prausnitzii* relative abundance in the intestine of the subjects suggested that the newly secured *Faecalibacterium prausnitzii* KBL1026 and KBL1027 strains belongs to the same species, but the intestinal microflora environment of the derived subjects is different, and therefore the functionality of the strains may be different.

### Example 3. Analysis of inflammatory disease improvement effect using animal model

### (1) Animal model construction and diet

To confirm the inflammatory disease improvement effect in vivo by single strain administration of *Faecalibacterium prausnitzii* KBL1026 or KBL1027 strain, an animal experiment was conducted (Seoul National University IACUC approval number: SNU-160602-9). As an example of inflammatory disease, an inflammatory bowel disease animal model was used to use in the experiment. To compare the inflammatory disease improvement efficacy of the strain, *Faecalibacterium prausnitzii* DSM17677 strain which belonged to the same species and was known to have the anti-inflammatory efficacy was adopted from Germany culture collection (DSMZ) to use as a control group.

Specifically, to construct a colitis mouse model, C57BL/6 7~8-week-old female mice were divided to colonies of 8 mice each, and then 2.5% dextran sulfate sodium (DSS) was dissolved in drinking water and they were made to drink it for 5 days in total to induce acute enteritis. To the normal control group, drinking water without DSS was provided.

The *Faecalibacterium prausnitzii* KBL1026, KBL1027, or DSM17677 strain was cultured in a YBHI liquid medium under an anaerobic condition at 37°C to reach an exponential phase, respectively, and then the supernatant was removed and it was diluted in PBS to be 2×10⁸ CFU/ml, and then from one week before the start of DSS supply until the end of the experiment, 200 µl of each of the diluted strain was orally administered to mice every day for colonization. To the normal control group, 200 µl of each of PBS was orally administered.

### (2) Body weight change analysis

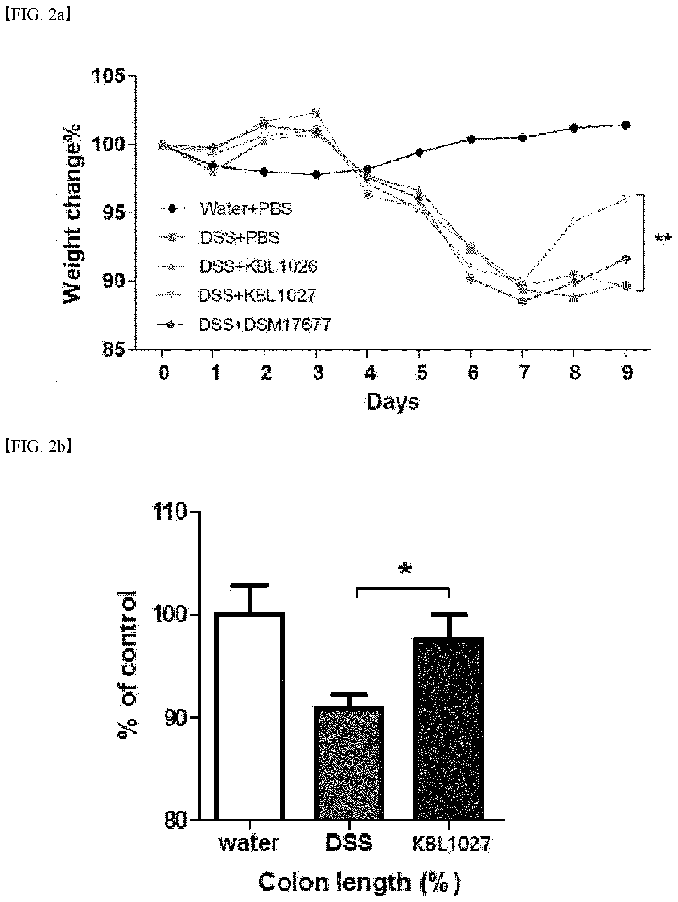

DSS supply was stopped in 5 days (Day 5 morning), and for 9 days from the day of the start of DSS supply (Day 0), the body weight change of mice was measured daily and was shown in FIG. 2a. FIG. 2a is a drawing which shows the change of the mouse body weight according to acute enteritis. As shown in FIG. 2a, in the DSS control group (DSS+PBS) compared to the normal control group not administered with DSS (Water+PBS), a significant decrease of the body weight was shown, thereby confirming construction of the colitis mouse model. In addition, in the KBL1027 administration group (DSS+KBL1027), compared to the DSS control group (DSS+PBS), a significantly improved effect in the body weight loss was confirmed. On the other hand, in the KBL1026 administration group and DSM17677 administration group, compared to the DSS control group (DSS+PBS), there was no significant weight change.

### (3) Intestine length analysis

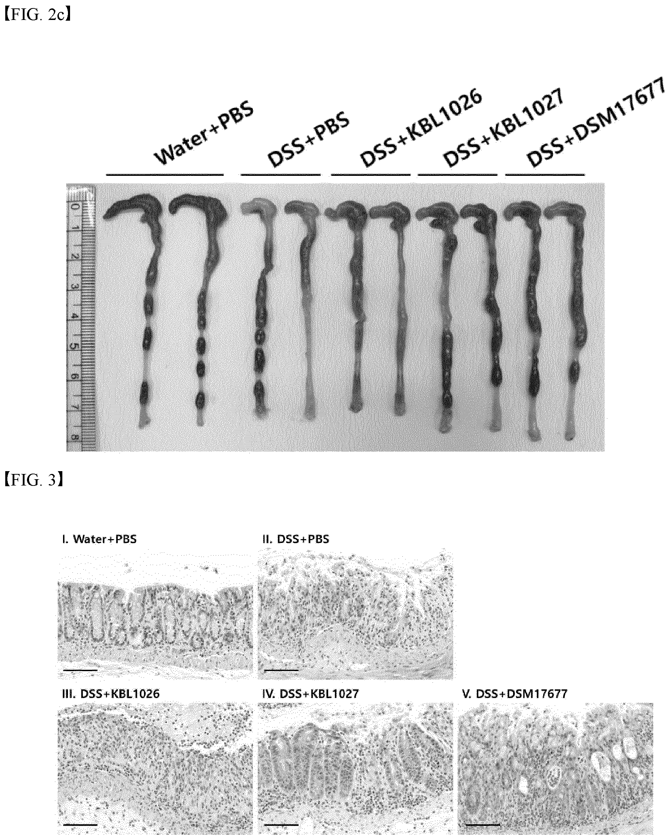

On the 9^{th} day after the start of DSS supply, the experiment was terminated and the mice were autopsied to measure the change in the intestinal length, and it was shown in FIG. 2b. As shown in FIG. 2b, it could be confirmed that the width of decrease of the large intestine length was significantly improved in the KBL1027 administration group compared to the DSS control group. FIG. 2c is a drawing which shows the process of measuring the intestine length after mouse autopsy.

### (4) Histopathological analysis of intestine

In addition, Hematoxylin&Eosin (H&E) staining was conducted to observe the histopathological change of the large intestine caused by *Faecalibacterium prausnitzii* single strain administration. Specifically, after autopsy, the distal part of the large intestine was fixed in 10% neutral formalin solution, and then paraffin tissue specimens were sectioned to a thickness of 5 µm and stained with H&E reagent and observed with an optical microscope. The H&E staining result was shown in FIG. 3, and infiltration of inflammatory cells in the large intestine tissue and significant destruction of the mucosal tissue were observed in DSS control group (DSS+PBS) compared to the normal control group (Water+PBS). On the other hand, it could be histologically confirmed that inflammation was alleviated in KBL1027 administration group (DSS+KBL1027) compared to DSS control group (DSS+PBS).

### (5) Analysis of effect of improving inflammatory disease in vivo using marker genes

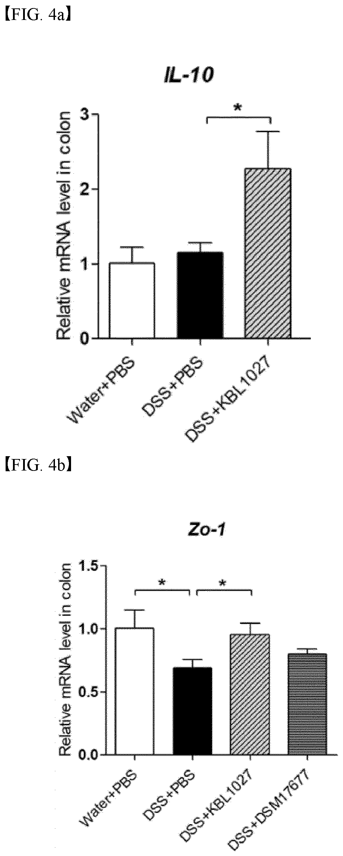

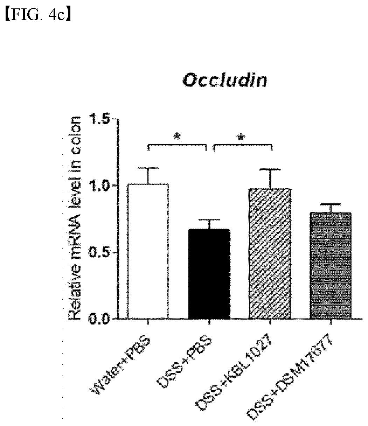

For analysis of immunocytes of the tissue, at the end of the experiment of Example 2, the intestinal tissue of mice was obtained and preserved in RNAlater (Thermo Fisher Scientific) solution, and kept frozen at -81 °C, and to extract RNA, easy-spin total RNA extraction kit (Intron) was used. The extracted RNA was synthesized with cDNA using high-capacity RNA-to-cDNA kit (Applied biosystems) in equal quantity immediately, and then the gene expression was analyzed using roter-gene SYBR green PCR kit (Qiagen). Using IL-10 known as anti-inflammatory cytokine and Zo-1, and Occludin genes related to tight junction between intestinal epithelial cells as a target, primers of Table 4 were used, and the expression was corrected with a HPRT housekeeping gene, and the result of analyzing the gene expression was shown in FIG. 4a (IL-10), FIG. 4b (Zo-1), and FIG. 4c (Occludin)

**[Table 4]**

| Classification | Target gene | Sequence (5'→3') | SEQ ID NO: |
|---|---|---|---|
| Forward | *HPRT* | TTATGGACAGGACTGAAAGAC | 7 |
| Reverse | *HPRT* | GCTTTAATGTAATCCAGCAGGT | 8 |
| Forward | *IL-10* | ATAACTGCACCCACTTCCCA | 9 |
| Reverse | *IL-10* | TCATTTCCGATAAGGCTTGG | 10 |
| Forward | *Zo-1* | ACCCGAAACTGATGCTGTGGATAG | 11 |
| Reverse | *Zo-1* | AAATGGCCGGGCAGAACTTGTGTA | 12 |
| Forward | *Occludin* | GGAGGACTGGGTCAGGGAATA | 13 |
| Reverse | *Occludin* | CGTCGTCTAGTTCTGCCTGT | 14 |

As shown in FIG. 4a, the expression of anti-inflammatory cytokine, *IL-10* gene was significantly increased in KBL1027 administration group (DSS+KBL1027) compared to DSS control group (DSS+PBS). This means that *Faecalibacterium prausnitzii* KBL1027 strain contributes to the effect of improving inflammatory disease in vivo through immunoregulation which induces IL-10 production.

According to the result of FIG. 4b and FIG. 4c, in case of *Zo-1* and *Occludin* related to tight junction between intestinal epithelial cells, both genes showed a significant decrease in expression in DSS control group (DSS+PBS) compared to the normal control group. On the other hand, the expression of both *Zo-1* and *Occludin* was significantly increased in KBL1027 administration group (DSS+KBL1027) compared to DSS control group (DSS+PBS). This means that KBL1027 strain administration increased the expression of *Zo-1* and *Occludin* and strengthened the tight junction between intestinal epithelial cells, thereby helping alleviation of inflammatory disease in vivo.

### Example 4. Analysis of intestinal microflora change using animal model

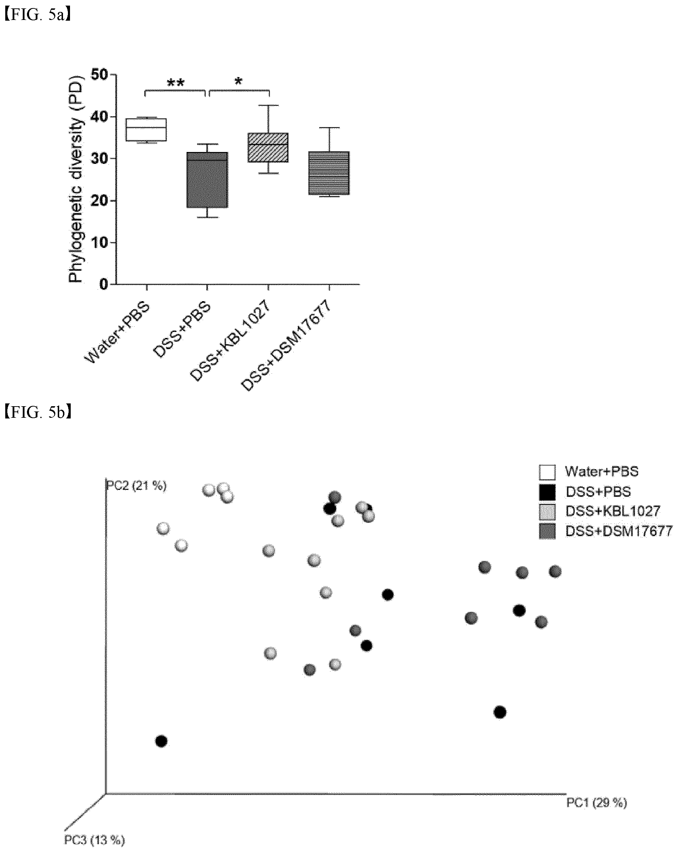

To analyze the change in the intestinal microflora induced by acute enteritis by DSS and colonization of a single strain, at the end of the experiment of Example 3, feces of mice were obtained and kept frozen at -81 °C. From the frozen sample, according to the method of Example 1, total bacterial genomic DNA was extracted and high-capacity sequence data were produced using V4 region of 16S rRNA genes of bacteria as a target. Using QIIME pipeline, the total genetic information of intestinal bacteria was confirmed and the structure of the microflora in mouse feces was identified, and univariate analysis (LefSE) according to the group was carried out and the result was shown in FIG. 5a to FIG. 5c.

The result of confirming the change in the diversity of intestinal microflora according to the group for the intestinal microflora based on 16S rRNA with Faith's Phylogenetic diversity index was shown in FIG. 5a, and it was confirmed that the diversity of the intestinal microflora was significantly reduced in DSS control group (DSS+PBS) compared to normal control group (Water+PBS). This suggests that according to acute enteritis caused by DSS, the decrease of the diversity of beneficial bacteria and dominance of potential harmful bacteria may negatively affect intestinal health. On the other hand, it was confirmed that the diversity of the intestinal microflora was significantly increased in *Faecalibacterium prausnitzii* KBL1027 administration group (DSS+KBL1027) compared to DSS control group (DSS+PBS), and there was no significance in DSM17677 administration group (DSS+ DSM17677). This suggests that even with the same *Faecalibacterium prausnitzii* strain, the effect on the intestinal microflora may be different and administration of the *Faecalibacterium prausnitzii* KBL1027 strain may recover the diversity of the intestinal microflora, thereby positively affecting the intestinal health.

The result of performing the major component analysis through weighted UniFrac distance of the intestinal microflora analyzed on the basis of 16S rRNA was shown in FIG. 5b, and it was confirmed that DSS control group had the intestinal microflora structure very different from the normal control group by PCA plot. On the other hand, it was confirmed that KBL1027 administration group had the structure of the intestinal microflora between the DSS control group and normal control group. This suggests that KBL1027 strain administration changes the species constituting the intestinal microflora, thereby modulating the structure.

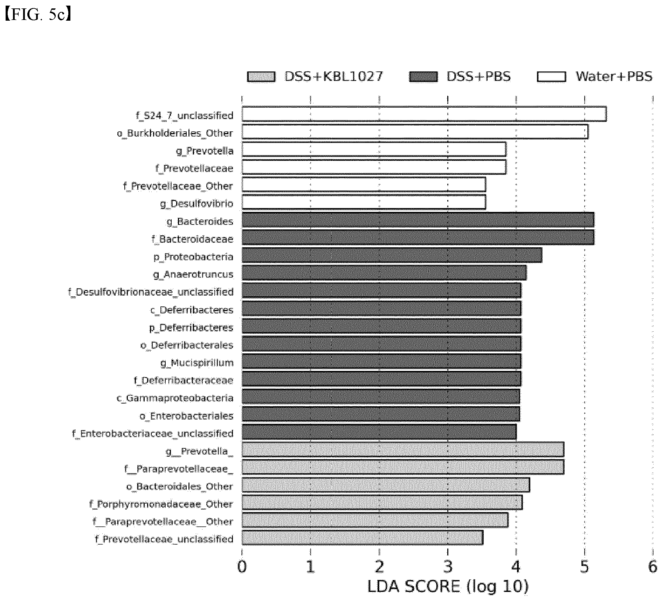

The result of conducting univariate analysis (LefSE) to analyze which intestinal microflora was changed by KBL1027 strain administration was shown in FIG. 5c. In the DSS control group, the dominance of genus *Bacteroides* and a representative beneficial bacterium, Proteobacteria of which cell wall is composed of lipopolysaccharides (LPS). This means that KBL1027 strain administration helped alleviation of colitis by changing this intestinal microflora.

### Example 5. Anti-inflammation test of F. prausnitzii KBL1027 strain derived substance

### (1) Observation of extracellular polysaccharide specific to KBL1027 strain

To confirm the specificity between *Faecalibacterium prausnitzii* strains (strain specificity), the three-dimensional surface shape of bacteria samples was observed using a scanning electron microscope (SEM).

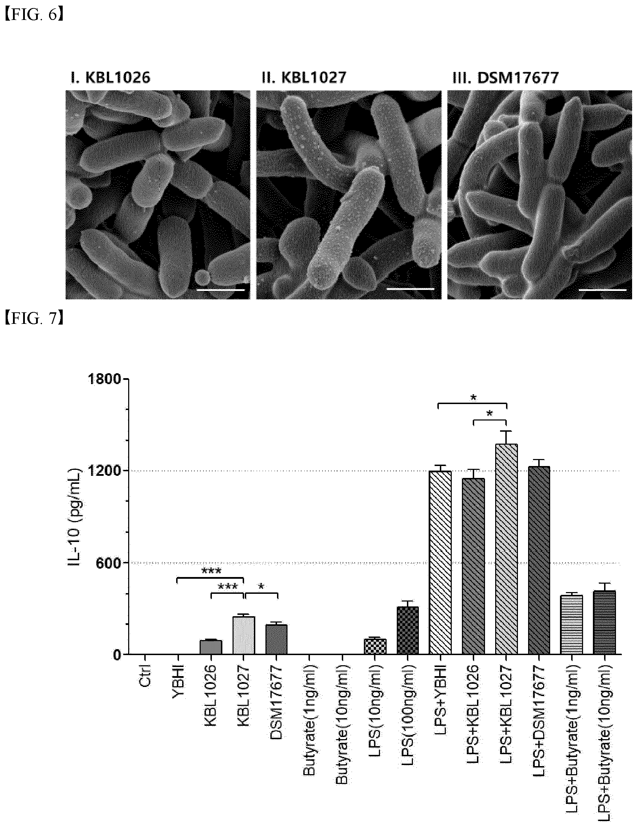

Specifically, samples obtained by culturing *Faecalibacterium prausnitzii* KBL1026, KBL1027, and DSM17677 strains in a YBHI medium under an anaerobic condition at 37 °C for 48 hours were under a pretreatment process of fixation, ethanol dehydration and drying at a critical point using karnovsky's solution and 2 % osmium tetroxide. Then, they were fixed on a stub and coated with platinum and then observed using a scanning electron microscope (Carl Zeiss). The observation photograph was shown in FIG. 6, and it was confirmed that KBL1027 produced an extracellular polysaccharide substance on the cell surface strain-specifically compared to KBL1026 and DSM17677 strains. On the other hand, in case of KBL1026, a form similar to the previously reported DSM17677 was observed. As such, the substance produced specifically by KBL1027 strain was confirmed through observation of the bacterial cell surface, and this strain specificity suggests that the excellent function of alleviating inflammatory disease of KBL1027 strain may be derived, compared to other strains belonging to *Faecalibacterium prausnitzii* species.

### (2) Anti-inflammatory efficacy by extracellular polysaccharide of KBL1027 strain

For verification of the immunoregulatory effect by the substance produced by the *Faecalibacterium prausnitzii* KBL1027 strain, bone-marrow derived macrophage (BMDM) of mice were isolated and treated with culture solution of *Faecalibacterium prausnitzii* KBL1026, KBL1027, and DSM17677 strains to confirm the ability to produce IL-10, a cytokine involved in the anti-inflammatory response.

Specifically, to obtain culture solution of the strain, the *Faecalibacterium prausnitzii* KBL1026, KBL1027, and DSM17677 strains were cultured in 500 ml of YBHI medium under an anaerobic condition at 37 °C for 48 hours to obtain supernatant by centrifugation and 0.45 µm filtering was progressed. As a negative control group, a YBHI medium not inoculated with the strain was used, and the secured medium and supernatant were prepared by lyophilizing after keeping frozen at -81 °C and diluting in PBS with the same amount to be 100 mg/ml.

To obtain BMDM cells of mice, bone-marrow of C57BL/6 mice was isolated and cultured in a medium for BMDM that 10% fetal bovine serum (FBS), 15% L292 cell culture solution and 1% penicillin/streptomycin antibiotics were added to a DMEM medium under the conditions of 37 °C, 5 % CO₂ using an incubator for 7 days. L292 cells were purchased from Korean Cell Line Bank.

After aliquoting 2×10⁴ cells into each well of a 96-well plate and attaching them for 24 hours, the negative control group YBHI medium and supernatant of the *Faecalibacterium prausnitzii* KBL1026, KBL1027, and DSM17677 strains were added to the culture solution at a concentration of 1 mg/ml and replaced. In addition, to confirm the response upon stimulation of the inflammatory response induced by LPS, LPS was added at a concentration of 100 ng/ml and then the supernatant of each strain was added in the same amount. As a positive control group, LPS was treated at a concentration of 10 ng/ml, 100 ng/ml, and for comparison with a butyrate metabolite produced by *Faecalibacterium prausnitzii,* sodium butyrate (sigma Aldrich) was treated with 1 ng/ml, 10 ng/ml. Then, in 24 hours, the culture solution was collected and kept frozen at -81 °C, and later, the amount of IL-10 cytokine was measured using mouse IL-10 ELISA kit (Thermo Fisher Scientific) according to the manufacturer's method.

The result was shown in FIG. 7, it was confirmed that in BMDM cells of mice, the culture solution of the *Faecalibacterium prausnitzii* KBL1027 strain most significantly induced production of anti-inflammatory cytokine, IL-10, compared to the negative control group, YBHI medium, and the culture solution of KBL1026 and DSM17677 strains. In addition, even in the situation where the inflammatory response through LPS was stimulated, IL-10 production was significantly induced than the negative control group, YBHI medium and the culture solution of KBL1026 strain. Such an experimental result means that the substance specifically produced by the *Faecalibacterium prausnitzii* KBL1027 strain induced production of anti-inflammatory cytokine IL-10.

### Example 6. Analysis of comparative genomics of F. prausnitzii KBL1027 strain

To confirm the specificity between *Faecalibacterium prausnitzii* strains (strain specificity), analysis of comparative genomics was conducted. To secure the genomic information of bacteria, KBL1026, and KBL1027 strains were cultured in a YBHI medium at an anaerobic condition at 37 °C to extract genomic DNA of strains according to the method of Example 1. In case of KBL1026, high-capacity sequence data were produced using MiSeq (Illumina) and assembled according to the A5-MiSeq pipeline, and the result was shown in Table 5 below. In case of KBL1027, the total information of genome (complete genome) was secured using RSII (PacBio). The genome information of the DSM17677 was secured from NCBI and used for analysis of comparative genomics (accession number: GCA_000162015.1). For comparison of average nucleotide identity (ANI) of genome, it was calculated by JSpeciesWS (http://j species.ribohost.com/jspeciesws/#analyse) and the summary and comparison of genome information of the secured strain were shown in Table 5. The ANI value of the KBL1027 strain and KBL1026 strain was 96.13 %, and on the other hand, it was calculated as 83.25 % with the DSM17677 strain. This means that the KBL1027 strain has a higher genome sequence similarity with the KBL1026 strain than the previously known DSM17677 strain.

**[Table 5]**

| Classification | KBL 1026 | KBL1027 | DSM17677 |
|---|---|---|---|
| Size (bp) | 3,105,066 | 3,124,218 | 3,090,349 |
| GC content (%) | 56.2 | 56.1 | 56.4 |
| Contig N50 (bp) | 330,297 | - | 63,061 |
| Number of scaffolds | 36 | 1 | 20 |
| Number of coding sequences | 3,193 | 3176 | 3,298 |
| Number of RNAs | 71 | 83 | 79 |
| ANI (%) with KBL 1027 | 96.13 | 100 | 83.25 |

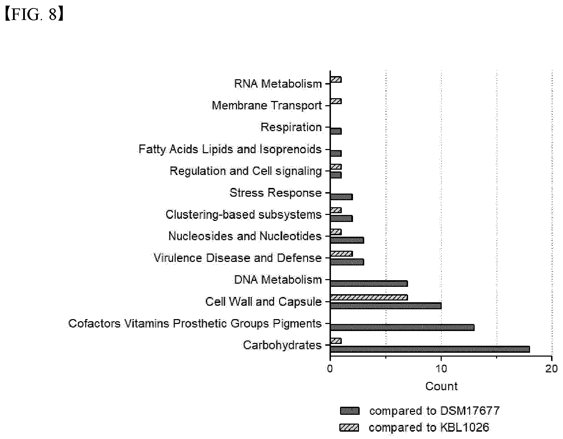

Based on the secured genome information of the *Faecalibacterium prausnitzii* strain, annotation was progressed according to the RASTtk pipeline using RAST (https://rast.nmpdr.org/) and analysis of comparative genomics was conducted. Genes specifically present in the genome of the KBL1027 strain compared to the DSM17677 strain were 61 in total, and genes specifically present in the genome of the KBL1027 strain compared to the KBL1026 strain were 15 in total. This is interpreted that more genes are specifically present because the KBL1027 strain has a lower ANI value than the DSM17677 strain. The number of genes specifically present only in the genome of the KBL1027 strain when compared with the two comparative strains, KBL1026 strain and DSM17677 strain, was shown in FIG. 8 according to their functional characteristics.

Among them, in case of the function of Cell Wall and Capsule involved in production of the extracellular polysaccharide substance defined in Example 5, it was confirmed that 7 genes compared to the KBL1026 strain and 10 genes compared to the DSM17677 strain were specifically present.

Next, 13 genes (following (1) to (13)) which were not present in both KBL1026 strain and DSM17677 strain and were present specifically only in the genome of the KBL1027 strain were confirmed, and among them, 7 (following (1) to (7)) belonged to the function of Cell Wall and Capsule:
(1) Exopolysaccharide biosynthesis glycosyltransferase **EpsF** (EC 2.4.1.-)
(2) Tyrosine-protein kinase **EpsD** (EC 2.7.10.2)
(3) Alpha-D-GlcNAc alpha-1,2-L-rhamnosyltransferase (EC 2.4.1.-) **rgpA**
(4) Alpha-L-Rha alpha-1,3-L-rhamnosyltransferase (EC 2.4.1.-) **rgpB**
(5) capsular polysaccharide biosynthesis protein
(6) Lipoprotein releasing system ATP-binding protein **LolD**
(7) Putative N-acetylgalactosaminyl-diphosphoundecaprenol glucuronosyltransferase **TuaG**
(8) Predicted transcriptional regulator of N-Acetylglucosamine utilization, GntR family **NagQ**
(9) Membrane-bound lytic murein transglycosylase B (EC 3.2.1.-)
(10) Inosine-uridine preferring nucleoside hydrolase (EC 3.2.2.1)
(11) LysR family transcriptional regulator **YnfL**
(12) Cation efflux system protein **CusA**
(13) Cobalt-zinc-cadmium resistance protein **CzcA**

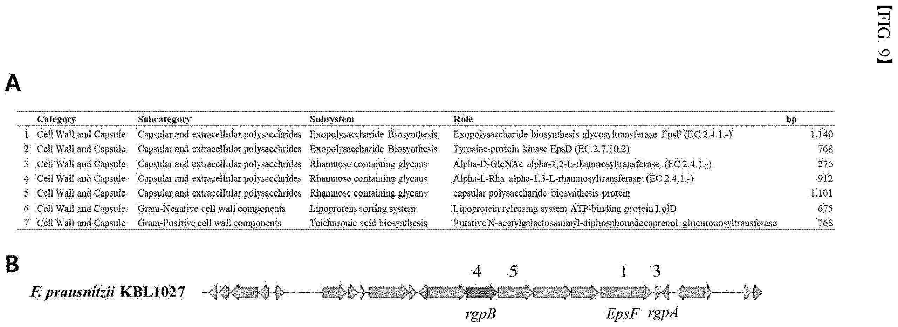

Genes belonging to the Cell Wall and Capsule function specifically present in the genome of the *Faecalibacterium prausnitzii* KBL1027 strain were shown in FIG. 9, and it was confirmed that the KBL1027 strain specific genes performed the function of exopolysaccharide biosynthesis and rhamnose containing glycans and formed a cluster (B of FIG. 9). B of FIG. 9 is a drawing which shows the result of confirming the cluster of Nos. 1, 3, 4 and 5 genes in A of FIG. 9 on the genomic sequence of the *Faecalibacterium prausnitzii* KBL1027 strain. Such result of the comparative genomic analysis means that the *Faecalibacterium prausnitzii* KBL1027 strain specifically produces an extracellular polysaccharide substance.

### [Accession Number]

Depository Authority Name : Korea Research Institute of Bioscience and Biotechnology
Accession Number : KCTC14230BP
Accession Date : 20200707
Depository Authority Name : Korea Research Institute of Bioscience and Biotechnology
Accession Number : KCTC14231BP
Accession Date : 20200707

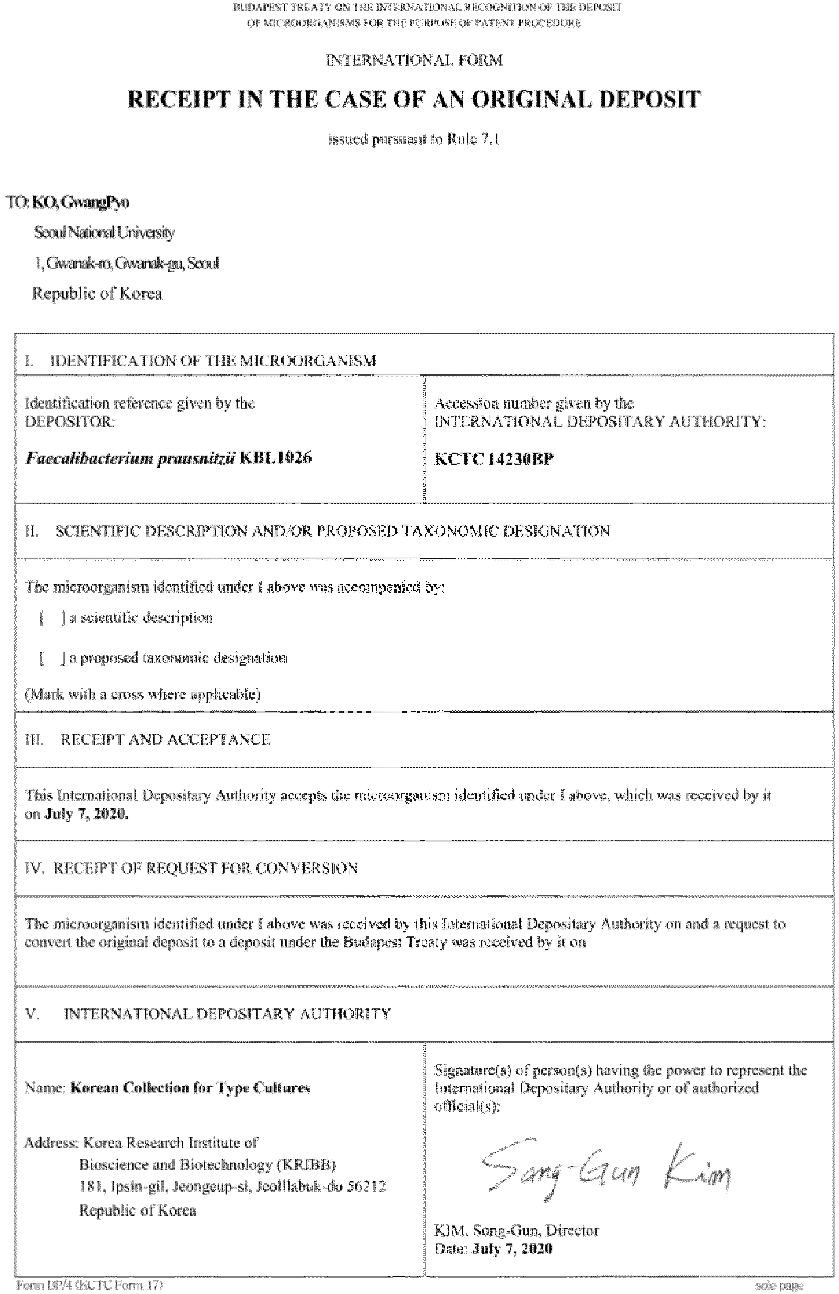
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KO, GwangPyo
Seoul National University
1, Gwanak-ro, Gwanak-gu, Seoul
Republic of Korea

I. IDENTIFICATION OF THE MICROORGANISM
Identification reference given by the
DEPOSITOR:
Faecalibacterium prausnitzii KBL1026 | Accession number given by the
INTERNATIONAL DEPOSITARY AUTHORITY:
KCTC 14230BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on July 7, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: July 7, 2020
Form BP/4 (KCTC Form 17) sole page

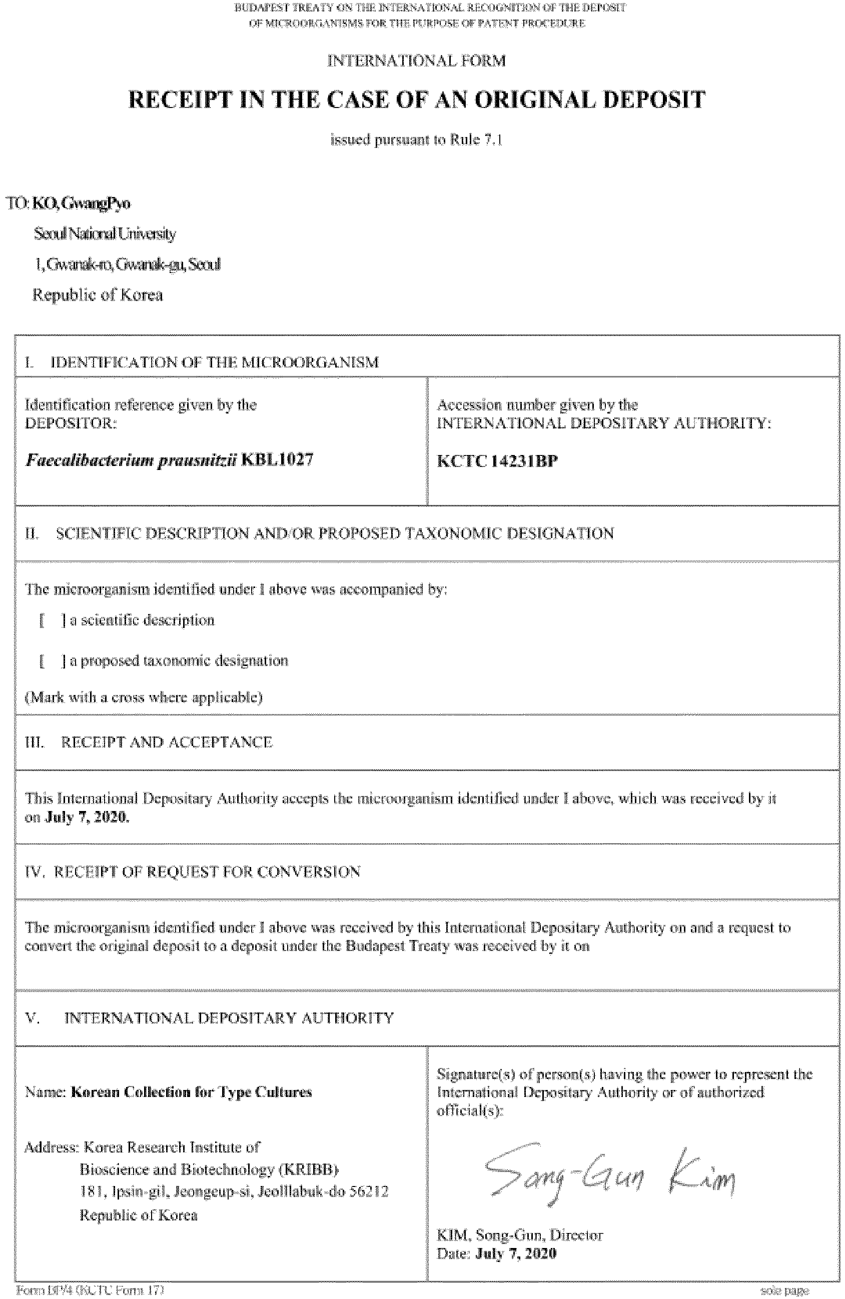
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KO, GwangPyo
Seoul National University
1, Gwanak-ro, Gwanak-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Faecalibacterium prausnitzii KBL1027 | KCTC 14231BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on July 7, 2020.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures | Signature(s) of person(s) having the power to represent the
International Depositary Authority or of authorized
official(s):
Address: Korea Research Institute of
Bioscience and Biotechnology (KRIBB)
181, Ipsin-gil, Jeongeup-si, Jeollabuk-do 56212
Republic of Korea |
KIM, Song-Gun, Director
Date: July 7, 2020

Form BP/4 (KCTC Form 17) sole page

## Claims

1. *Faecalibacterium prausnitzii* KBL1027 strain having accession number KCTC14231BP.

2. The strain according to claim 1, wherein the strain has a 16S rRNA sequence represented by SEQ ID NO: 4.

3. The strain according to claim 1, wherein the strain produces extracellular polysaccharide.

4. The strain according to claim 1, wherein the strain comprises gene functioning in cell wall and capsule.

5. The strain according to claim 4, wherein the gene functioning in cell wall and capsule performs the function of exopolysaccharide biosynthesis or rhamnose containing glycans.

6. The strain according to claim 4, wherein the gene functioning in cell wall and capsule comprises one or more genes selected from the group consisting of the following (1) to (7):
(1) Exopolysaccharide biosynthesis glycosyltransferase,
(2) Tyrosine-protein kinase,
(3) Alpha-D-GlcNAc alpha-1,2-L-rhamnosyltransferase,
(4) Alpha-L-Rha alpha-1,3-L-rhamnosyltransferase,
(5) capsular polysaccharide biosynthesis protein,
(6) Lipoprotein releasing system ATP-binding protein,
(7) Putative N-acetylgalactosaminyl-diphosphoundecaprenol glucuronosyltransferase.

7. The strain according to claim 1, wherein the strain comprises one or more genes selected from the group consisting of the following (1) to (13):
(1) Exopolysaccharide biosynthesis glycosyltransferase,
(2) Tyrosine-protein kinase,
(3) Alpha-D-GlcNAc alpha-1,2-L-rhamnosyltransferase,
(4) Alpha-L-Rha alpha-1,3-L-rhamnosyltransferase,
(5) capsular polysaccharide biosynthesis protein,
(6) Lipoprotein releasing system ATP-binding protein,
(7) Putative N-acetylgalactosaminyl-diphosphoundecaprenol glucuronosyltransferase,
(8) Predicted transcriptional regulator of N-Acetylglucosamine utilization, GntR family,
(9) Membrane-bound lytic murein transglycosylase B,
(10) Inosine-uridine preferring nucleoside hydrolase,
(11) LysR family transcriptional regulator,
(12) Cation efflux system protein,
(13) Cobalt-zinc-cadmium resistance protein.

8. The strain according to claim 1, wherein the strain has a property of inducing anti-inflammatory cytokine production.

9. The strain according to claim 1, wherein the strain has a property of strengthening tight junction between intestinal epithelial cells.

10. The strain according to claim 1, wherein the strain has a property of improving the intestinal microbial balance.

11. The strain according to claim 10, wherein the improving the intestinal microbial balance is one or more selected from the group consisting of the following (1) to (5):
(1) inducing an increase in the diversity of intestinal microflora of an animal,
(2) improving imbalance of intestinal microflora of an animal,
(3) inducing an increase in the relative abundance of strains of genus Prevotella and/or family Paraprevotellaceae in intestinal microflora of an animal,
(4) preventing an increase in the relative abundance of harmful bacteria having a lipopolysaccharide cell wall in intestinal microflora of an animal,
(5) preventing an increase in the relative abundance of strains of phylum Proteobacteria, genus Bacteroides and/or phylum Proteobacteria in intestinal microflora of an animal.

12. The strain according to claim 1, wherein the strain has one or more characteristics selected from the group consisting of the following (1) to (2):
(1) preventing body weight loss due to inflammatory disease,
(2) preventing intestinal length reduction due to inflammatory disease.

13. A culture of the strain according to any one of claim 1 to claim 12, a lysate of the strain, an extract of the strain, an extract of the culture, an extract of the lysate, or an extracellular polysaccharide produced by the strain.

14. A composition for prevention, improvement or treatment of inflammatory disease, comprising one or more selected from the group consisting of the strain according to any one of claim 1 to claim 12, a culture of the strain, a lysate of the strain, an extract of the strain, an extract of the culture, an extract of the lysate, and an extracellular polysaccharide produced by the strain.

15. The composition according to claim 14, wherein the inflammatory disease is one or more selected from the group consisting of inflammatory bowel disease, inflammatory skin disease, inflammatory collagen-vascular disease, atopic dermatitis, allergic disease, autoimmune disease, autoimmune inflammatory disease, eczema, asthma, allergic asthma, bronchial asthma, rhinitis, allergic rhinitis, conjunctivitis, allergic conjunctivitis, food allergy, rheumatoid arthritis, rheumatoid fever, lupus, systemic scleroderma, psoriasis, psoriatic arthritis, asthma, Guilian-Barre syndrome, myasthenia gravis, dermatomyositis, multiple myositis, multiple sclerosis, autoimmune encephalomyelitis, polyarteritis nodosa, Hashimoto thyroiditis, temporal arteritis, pediatric diabetes, alopecia areata, pemphigus, aphthous stomatitis, autoimmune hemolytic anemia, Wegener granulomatosis, Sjogren syndrome, Addison's disease, Behcet's disease, edema, conjunctivitis, periodontitis, rhinitis, otitis media, chronic sinusitis, sore throat, tonsillitis, bronchitis, pneumonia, stomach ulcer, gastritis, colitis, gout, eczema, acne, contact dermatitis, seborrheic dermatitis, ankylosing myelitis, fibromyalgia, osteoarthritis, shoulder periarthritis, tendinitis, tenosynovitis, myositis, hepatitis, cystitis, nephritis, septicemia, angiitis, and bursitis.

16. The composition according to claim 15, wherein the inflammatory bowel disease is one or more selected from the group consisting of inflammatory colorectal disease, ulcerative colitis (UC), enteritis, irritable bowel syndrome (IBS) and Crohn's disease (CD).

17. The composition according to claim 14, wherein the prevention, improvement or treatment of inflammatory disease is caused by the extracellular polysaccharide of the strain.

18. A food composition for prevention or improvement of inflammatory disease, comprising one or more selected from the group consisting of the strain according to any one of claim 1 to claim 12, a culture of the strain, a lysate of the strain, an extract of the strain, an extract of the culture, an extract of the lysate, and an extracellular polysaccharide produced by the strain.

19. A probiotics composition for prevention or improvement of inflammatory disease, comprising one or more selected from the group consisting of the strain according to any one of claim 1 to claim 12, a culture of the strain, a lysate of the strain, an extract of the strain, an extract of the culture, an extract of the lysate, and an extracellular polysaccharide produced by the strain.

20. A composition for improving the intestinal microbial balance, comprising one or more selected from the group consisting of *Faecalibacterium prausnitzii* strain according to any one of claim 1 to claim 12, a culture of the strain, a lysate of the strain, an extract of the strain, an extract of the culture, an extract of the lysate, and an extracellular polysaccharide produced by the strain.

21. *A Faecalibacterium prausnitzii* strain having anti-inflammatory activity, a culture of the strain, a lysate of the strain, an extract of the strain, an extract of the culture, an extract of the lysate, or an extracellular polysaccharide produced by the strain,
wherein the strain is *Faecalibacterium prausnitzii* KBL1027 strain having accession number KCTC14231BP.

22. A *Faecalibacterium prausnitzii* strain producing a substance having anti-inflammatory activity, a culture of the strain, a lysate of the strain, an extract of the strain, an extract of the culture, an extract of the lysate, or an extracellular polysaccharide produced by the strain,
wherein the strain is *Faecalibacterium prausnitzii* KBL1027 strain having accession number KCTC14231BP.

23. The strain, the culture of the strain, the lysate of the strain, the extract of the strain, the extract of the culture, the extract of the lysate, or the extracellular polysaccharide produced by the strain according to claim 22, wherein the substance having anti-inflammatory activity is an extracellular polysaccharide.
